(19) Europäisches Patentamt
European Patent Office
Office Européen des brevets

(11) Veröffentlichungsnummer : 0 211 157 B1

# (12) EUROPÄISCHE PATENTSCHRIFT

(45) Veröffentlichungstag der Patentschrift : **02.01.91 Patentblatt 91/01**

(51) Int. Cl.[5] : **C07D 413/14, A61K 31/42**

(21) Anmeldenummer : **86106529.0**

(22) Anmeldetag : **14.05.86**

## (54) Isoxazolderivate, Verfahren zu ihrer Herstellung und diese enthaltende pharmazeutischen Präparate.

Die Akte enthält technische Angaben, die nach dem Eingang der Anmeldung eingereicht wurden und die nicht in dieser Patentschrift enthalten sind.

(30) Priorität : **17.05.85 AT 1493/85**

(43) Veröffentlichungstag der Anmeldung : **25.02.87 Patentblatt 87/09**

(45) Bekanntmachung des Hinweises auf die Patenterteilung : **02.01.91 Patentblatt 91/01**

(84) Benannte Vertragsstaaten : **AT BE CH DE FR GB IT LI LU NL SE**

(56) Entgegenhaltungen :
**EP-A- 0 111 345**
**EP-A- 0 137 242**

(73) Patentinhaber : **Hafslund Nycomed Pharma Aktiengesellschaft**
**St. Peter-Strasse 25**
**A-4021 Linz (AT)**
**BE CH DE FR GB IT LI LU NL SE AT**

(72) Erfinder : **Binder, Dieter, Dr.**
**Sieveringerstrasse 207**
**A-1190 Wien (AT)**
Erfinder : **Rovenszky, Franz, Dr., Dipl.-Ing.**
**Lagerhausstrasse 5/8**
**A-2460 Bruck a.d. Leitha (AT)**

(74) Vertreter : **Kunz, Ekkehard, Dr.**
**Chemie Holding AG Patentwesen St. Peter-Strasse 25**
**A-4021 Linz (AT)**

EP 0 211 157 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue, antiviral wirksame substituierte Isoxazolderivate, ein Verfahren zu ihrer Herstellung, pharmazeutische Präparate, die diese Verbindungen enthalten, sowie ihre Verwendung.

In den europäischen Schriften EP-A 0111 345 und EP-A 0137 242 werden Phenyl-aliphatische Isoxazolderivate beschrieben, die antiviral wirksam sind.

Es wurden nun Thieno-aliphatische Isoxazolderivate gefunden, die ebenso antivirale Aktivität besitzen.

Gegenstand der Erfindung sind daher neue substituierte Isoxazolderivate der allgemeinen Formel (I)

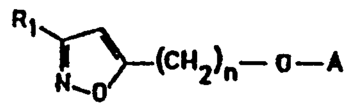

I

in der
R1 (C1 - C4)-Alkyl,
n eine ganze Zahl 6, 7 oder 8,
A eine Gruppe der allgemeinen Formeln (II) oder (III)

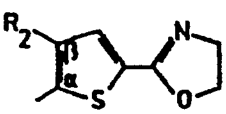

II

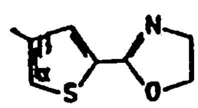

III

und
$R_2$ Wasserstoff oder Methtyl bedeuten,
ein Verfahren zu ihrer Herstellung, pharmazeutische Präparate, die diese Verbindungen enthalten, sowie ihre Verwendung.

Der in dieser Beschreibung verwendete Ausdruck "(C1 - C4)-Alkyl" bezeichnet geradkettige oder verzweigte Kohlenwasserstoffgruppen mit 1 bis 4 Kohlenstoffatomen, wie z.B. Methtyl, Ethyl, Propyl, Isopropyl, Butyl, tert. Butyl.

In einer bevorzugten Gruppe von Verbindungen der Formel (I) bedeutet R1 Methyl oder Ethtyl, wobei Methyl besonders bevorzugt ist. R2 ist bevorzugt Wasserstoff und n steht bevorzugt für die Zahl 7.

Eine besonders bevorzugte Gruppe innerhalb der Verbindungen der allgemeinen Formel (I) ist jene, in der A eine 5-(4,5-Dihydro-2-oxazolyl)-thienylgruppe der Formel(II) bedeutet, die in alpha-Stellung zum Schwefelatom mit dem Isoxazolyl-alkyloxyrest verbunden ist.

Besonders bevorzugte Einzelverbindungen sind 5-(7-(5-(4,5-Dihydro-2-oxazolyl)-2-thienyl)oxyheptyl)-3-methyl-isoxazol und 5(7-(2-(4,5-Dihydro-2-oxazolyl)-4-thienyl)oxyheptyl)-3-methyl-isoxazol.

Die Isoxazolderivate der allgemeinen Formel I können erfindungsgemäß dadurch hergestellt werden, daß man eine Verbindung der allgemeinen Formel (IV)

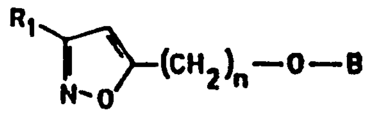

IV

in der $R_1$ und n wie oben definiert sind,
B eine Gruppe der allgemeinen Formeln (V) oder (VI)

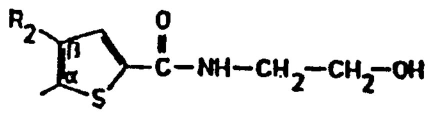

V

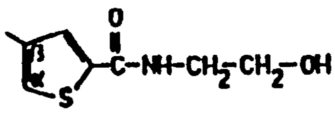

VI

bedeutet und

$R_2$ wie oben definiert ist, durch Behandeln mit einem wasserentziehenden Reagens unter Bildung des Oxazolrings cyclisiert.

Die erfindungsgemäße Umsetzung kann in An- oder Abwesenheit eines inerten organischen Lösungsmittels erfolgen.

Wird die Umsetzung in Anwesenheit eines Lösungsmittels durchgeführt, eignen sich als solche beispielsweise Kohlenwasserstoffe, wie Benzol, Toluol, Xylol, halogenierte Kohlenwasserstoffe, wie Chloroform, Chlorbenzol, Methylenchlorid, Tetrachlorkohlenstoff, Ether, wie Dioxan, Tetrahydrofuran, Dimethylformamid oder Mischungen solcher Lösungsmittel. Als wasserentziehende Mittel kommen dabei die für solche Cyclisierungen üblicherweise verwendeten Reagenzien in Frage, beispielsweise Phosphoroxychlorid, Phosphorpentachlorid oder Thionylchlorid. Das wasserentziehende Reagens kann hiezu in äquivalenten Mengen oder in einem geringen Überschuß, beispielsweise in Mengen von 1,1 bis 3 Mol pro Mol der Verbindung der Formel (IV), eingesetzt werden. Die Umsetzung erfolgt bei -20°C bis +10°C, vorzugsweise bei -5°C bis + 5°C.

In einer besonders bevorzugten Ausführungsform des erfindungsgemäßen Verfahrens werden die Verbindungen der allgemeinen Formel(IV) in Abwesenheit eines gesonderten Lösungsmittels durch Behandeln mit einem Überschuß eines flüssigen, wasserentziehenden Reagens, welches zugleich als Lösungsmittel dient, bei -30°C bis + 10°C, vorzugsweise -5°C bis +5° C, ganz besonders bevorzugt im Eisbad bei etwa 0°C cyclisiert. Geeignete Reagenzien für diesen Zweck sind wiederum Phosphoroxychlorid oder Thionylchlorid, wobei sich die Verwendung von Thionylchlorid als ganz besonders vorteilhaft erwiesen hat.

Die für das erfindungsgemäße Verfahren verwendeten Ausgangsverbindungen der allgemeinen Formel (IV) können ausgehend von bekannten Produkten in an sich bekannter Weise hergestellt werden. Insbesondere können sie gemäß dem folgenden Reaktionsschema und den spezifischen Angaben in den Beispielen synthetisiert werden.

Im folgenden Reaktionsschema haben $R_1$ und n die gleiche Bedeutung wie oben definiert. Gemäß der im Reaktionsschema gezeigten Reaktionsfolge können sowohl Verbindungen der allgemeinen Formel (IV), in denen B eine Gruppe der Formel (V), als auch Verbindungen der allgemeinen Formel (IV), in denen B eine Gruppe der Formel (VI) bedeutet, synthetisiert werden. Dementsprechend ist in den Formeln (X), (XI), (XIIa) und (XIII) des Reaktionsschemas der Hydroxy- bzw. Isoxazolyl-alkyloxyrest entweder in der alpha- oder beta-Stellung und, falls dieser die alpha-Stellung einnimmt, ein gegebenenfalls vorhandener Rest $R_2$ in der beta-Stellung mit der Thienylgruppe verbunden.

## Reaktionsschema

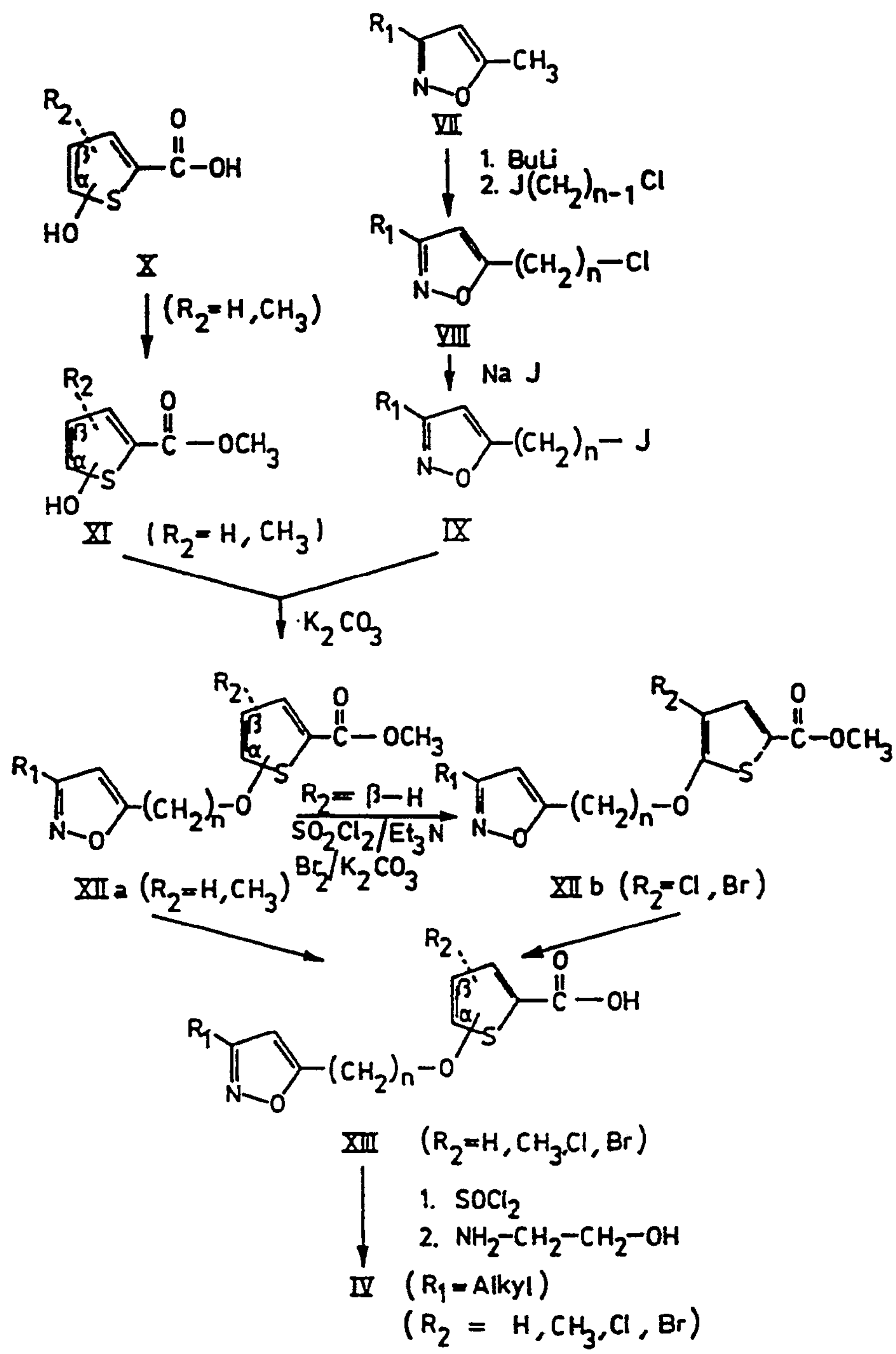


Die Verbindungen der allgemeinen Formel (I) haben eine antiinfektive, insbesondere eine ausgeprägte antivirale Wirkung. Diese wertvollen pharmakologischen Eigenschaften können in vitro und in vivo unter Verwendung von Standardmethoden bestimmt werden. Dabei zeigen die Verbindungen der allgemeinen Formel (I) insbesondere gegen verschiedene Typen von Enteroviren, Picornaviren und Rhinoviren hervorragende Wirkung und können daher in der Humanmedizin zur Behandlung und Prophylaxe von Viruserkrankungen eingesetzt werden.

Zur Untersuchung der antiviralen Eigenschaften wurde folgende Testmethode verwendet :

In Mikrotiterplatten mit flachem Boden wurden serielle Dreifachverdünnungen von Lösungen der zu untersuchenden Substanzen in MEM (minimum essential medium) hergestellt. Gleiche Volumina der jeweiligen Virusverdünnungen in MEM und Zellsuspension in MEM mit 15% FCS (foetales Kälberblutserum)

wurden zugesetzt. Die Zellkonzentration wurde dabei so gewählt, daß sich nach 1 - 2 Tagen ein konfluenter Zellrasen bildet. Die Virusverdünnungen wurden so eingestellt, daß ohne Zusatz einer Hemmsubstanz nach 3 - 4 Tagen ein vollständiger zytopathischer Effekt auftrat.

Als Kontrollen wurden Zellen (Zellkontrolle), Zellen mit Virus (Viruskontrolle) und Zellen mit den Testsubstanzen in verschiedener Konzentration (Toxizitätskontrolle) mitgeführt. Als minimale toxische Konzentration (MTK) wurde die Substanzkonzentration ermittelt, bei der eine noch geringere Zelldichte als in der Zellkontrolle beobachtet wurde.

Die Testsubstanzen wurden in Dimethylsulfoxid gelöst, in MEM verdünnt und mittels Ultraschall gut suspendiert.

Die erfindungsgemäßen Verbindungen wurden auf ihre antivirale Wirkung in einem repräsentativen Querschnitt an Rhinoviren der Typen B1-55, Echoviren Typ B-9 und Polioviren Typ 2 untersucht und dabei die minimale Hemmkonzentration (MHK in µg/ml) ermittelt.

In diesem als Standardmethode bekannten Virus-Hemm-Test wurden für Verbindungen der allgemeinen Formel (I) wie beispielsweise 5-(7-(5-(4,5-Dihydro-2oxazolyl)-2-thienyl)oxyheptyl)-3-methyl-isoxazol (Verbindung A) oder 5-(7-(2(4,5-Dihydro-2-oxazolyl)-4-thienyl)oxyheptyl)-3-methyl-isoxazol (VerbindungB) die in Tabelle 1 angegebenen MHK-Werte ermittelt.

Tabelle 1

Antivirale Wirkung in vitro von Verbindungen der allgemeinen Formel (I)

| Organismus Serotyp | | Minimale Hemmkonzentration (MHK, µg/ml) Verbindung A | Verbindung B |
|---|---|---|---|
| Rhino Typ | 3 | 0,03 | 0,1 |
| | 16 | 0,3 | 1,0 |
| | 26 | 0,3 | 1,0 |
| | 32 | 1,0 | 3,0 |
| | 37 | 0,03 | 0,03 |
| | 48 | 0,1 | 1,0 |
| | 55 | 0,3 | 0,3 |
| Echo Typ | B-9 | 0,03 | 1,0 |
| Polio Typ | 2 | 0,1 | 1,0 |
| MTK[1] | | 10 | 10 |

[1] Minimale toxische Konzentration

Gegenüber herkömmlichen Verbindungen mit antiviralen Eigenschaften haben die erfindungsgemäßen Verbindungen den Vorteil von verbesserten lipophilen Eigenschaften, die eine Überwindung der Blut-Hirnschranke ermöglichen.

Die Verbindungen der allgemeinen Formel (I) können als Heilmittel, z.B. in Form von pharmazeutischen Präparaten Verwendung finden, welche sie in Mischung mit einem für die enterale oder parenterale Applikation geeigneten pharmazeutischen, organischen oder anorganischen inerten Hilfs- und/oder Trägermaterial, wie z.B. pharmazeutisch unbedenklichen Lösungsmitteln, Gelatine, Gummi arabicum, Milchzucker, Stärke, Magnesiumstearat, Talk, pflanzliche Öle, Polyalkylenglykole oder Vaseline, enthalten.

Die pharmazeutischen Präparate können in fester Form, beispielsweise als Tabletten, Dragees, Suppositorien oder Kapseln, in halbfester Form, beispielsweise als Salben oder in flüssiger Form, beispielsweise als Lösungen, Suspensionen oder Emulsionen, vorliegen. Gegebenenfalls sind sie sterilisiert und enthalten Hilfsstoffe, wie Konservierungs-, Stabilisierungs-oder Emulgiermittel, Salze zur Veränderung des osmotischen Druckes und dergleichen.

Insbesondere können pharmazeutische Präparate die erfindungsgemäßen Verbindungen in Kombination mit anderen therapeutisch wertvollen Stoffen, beispielsweise mit anderen antiinfektiven oder antiviralen Wirkstoffen, enthalten. Mit diesen können die erfindungsgemäßen Verbindungen beispielsweise zusammen mit den oben angegegenen Hilfs- und/oder Trägerstoffen zu Kombinationspräparaten formuliert werden.

Die folgenden Beispiele erläutern die Erfindung näher :

Beispiel 1 :

5-(7-(5-(4,5-Dihydro-2-oxazolyl)-2-thienyl)oxyheptyl)-3-methyl-isoxazol

0,60 g (1,64 mmol) N-(2-Hydroxyethyl)-5-(7-(3-methyl-5-isoxazolyl)-heptyloxy)-2-thiophencarbonsäureamid (IV) werden in 2,5 ml Thionylchlorid bei 0°C eingetragen, 15 min bei 0°C gerührt und anschließend das überschüssige Thionylchlorid im Vakuum entfernt. Der Rückstand wird zwischen gesättigter Natriumhydrogencarbonatlösung und Ethylacetat verteilt. Es wird noch zweimal mit Ethylacetat extrahiert, die vereinigten organischen Phasen über Natriumsulfat/Aktivkohle getrocknet und eingedampft.

Das Rohprodukt (0,52 g gelbliche Kristalle) wird säulenchromatographisch gereinigt (1 :35, Kieselgel 60, Korngröße 0,040-0,063 ; Eluens : Ethylacetat : Petrolether = 3 :1).
Ausbeute : 0,29 g farblose Kristalle (50,7% d.Th.)
Fp = 69 - 70°C (Diisopropylether)

Das Ausgangsmaterial kann wie folgt hergestellt werden :

5-(7-Chlorheptyl)-3-methyl-isoxazol (VIII)

21,0 g (0,216 mol) 3,5-Dimethylisoxazol (VII, hergestellt nach C. Kashima et al., Bull.Chem.Soc.Jap.46, 310, 1973) werden in 200 ml abs. THF gelöst, die Lösung auf -80°C gekühlt und bei dieser Temperatur 160 ml n-Butyllithium (1,35 m Lösung in n-Hexan, 0,216 mol) innerhalb von 40 min zugetropft. Es wird noch 15 min bei einer Temperatur unter -75°C gerührt.

Das Reaktionsgemisch wird anschließend zu einer Lösung von 53,5 g (0,217 mol) 1-Jod-6-chlorhexan (hergestellt nach W.F.Huber, J.Am.Chem.Soc. 73, 2730, 1951) in 150 ml abs. THF so zugetropft, daß die Temperatur -60°C nicht übersteigt. Nach beendeter Zugabe wird noch 15 min. bei -60°C gerührt und auf Raumtemperatur erwärmen lassen.

Das Reaktionsgemisch wird zwischen Methylenchlorid und 0,2 n HCl verteilt, die wäßrige Phase noch dreimal mit Methylenchlorid extrahiert, die vereinigten organischen Phasen über Natriumsulfat getrocknet und eingedampft.

Das Rohprodukt (ca.45 g) wird portionsweise im Kugelrohr destilliert (Luftbadtemperatur 80°C / 0,2 mbar).
Ausbeute : 26,9 g, gelbliches Öl

5-(7-(3-Methyl-5-isoxazolyl)heptyloxy)-2-thiophencarbonsäure-methylester (XIIa)

9,3 g (43,1 mmol) 5-(7-Chlorheptyl)-3-methyl-isoxazol und 7,12 g (47,4 mmol) Natriumjodid werden in 60 ml abs. Aceton 24 Stunden unter Rückfluß erhitzt. Anschließend wird abgekühlt, ausgefallenes NaCl abgesaugt, mit wenig Aceton gewaschen und das Filtrat mit 7,16 g (45,3 mmol) 5-Hydroxy-2-thiophencarbonsäuremethylester (XI) und 13,1 g (94,8 mmol) Kaliumcarbonat versetzt. Es wird 2 Stunden unter Rückfluß erhitzt, abgekühlt und weitgehend eingedampft. Der Rückstand wird zwischen Wasser und Ether verteilt und die wäßrige Phase zweimal mit Ether extrahiert. Die vereinigten organischen Phasen werden mit wenig gesättigter Natriumhydrogensulfit-Lösung gewaschen, über Natriumsulfat / Aktivkohle getrocknet und eingedampft.

Das Rohprodukt (11,9 g ; 82% d.Th.) wird aus Diisopropylether umkristallisiert.
Ausbeute : 5,2 g hellrosa Kristalle (35,5% d.Th.)

Fp = 55-56°C (Diisopropylether)

5-(7-(3-Methyl-5-isoxazolyl)heptyloxy)-2-thiophencarbonsäure (XIII)

0,98 g (2,90 mmol) 5-(7-(3-Methyl-5-isoxazolyl)heptyloxy)-2-thiophencarbonsäuremethylester werden in 8 ml Ethanol und 4 ml Wasser unter Rückfluß erhitzt und in diese Lösung 0,18 g (3,19 mmol) KOH, gelöst in 6 ml Wasser und 4 ml Ethanol, in 10 min. zugetropft. Anschließend wird noch 2,5 Stunden unter Rückfluß erhitzt.

Nach dem Abkühlen wird größtenteils eingedampft, zwischen Wasser und Ether verteilt und die wäßrige Phase mit 2n HCl auf pH = 1,5 angesäuert. Es wird dreimal mit insgesamt 80 ml Ether extrahiert, die vereinigten organischen Phasen über Natriumsulfat/Aktivkohle getrocknet und eingedampft.
Ausbeute : 0,90 g farblose Kristalle (95,8% d.Th.)
Fp = 96-7°C (Diisopropylether)

N-(2-Hydroxyethyl)-5(7-(3-methyl-5-isoxazolyl)-heptyloxy)-2-thiophencarbonsäureamid (IV)

Zu 0,81 g (2,51 mmol) 5-(7-(3-Methyl-5-isoxazolyl)heptyloxy)-2-thio phencarbonsäure werden unter Kühlen und Rühren 2 ml Thionylchlorid langsam zugetropft, wobei eine klare Lösung entsteht. Es wird noch 30 min. bei Raumtemperatur gerührt und anschließend überschüssiges Thionylchlorid im Vakuum entfernt. Der Rückstand wird in 6 ml absolutem Methylenchlorid gelöst und bei einer Temperatur von 15°C eine Lösung von 0,34 g (5,51 mmol) Ethanolamin in 5 ml abs. Methylenchlorid zugetropft. Es wird noch 1 Stunde bei Raumtemperatur gerührt, etwas eingeengt und zwischen Wasser und Ethylacetat verteilt. Die wäßrige Phase wird noch einmal mit wenig Ethylacetat extrahiert, die vereinigten organischen Phasen mit Wasser gewaschen, über Natriumsulfat/Aktivkohle getrocknet und eingedampft.
Ausbeute : 0,81 g gelbliche Kristalle (88,2% d.Th.)
Fp = 107-110°C(Acetonitril)

Beispiel 2 :

5-(7-(2-(4,5-Dihydro-2-oxazolyl)-4-thienyl)oxyheptyl)-3-methyl-isoxazol

2,06 g (5,62 mmol) N-(2-Hydroxyethyl)-4-(7-(3-methyl-5-isoxazolyl)-heptyloxy)-2-thiophencarbonsäureamid (IV) werden in 5 ml Thionylchlorid bei 0°C eingetrage.n, 10 min bei 0°C gerührt und anschließend das überschüssige Thionylchlorid im Vakuum ohne zu erwärmen entfernt. Der Rückstand wird zwischen gesättigter Natriumhydrogencarbonatlösung und Ethylacetat verteilt. Es wird noch zweimal mit Ethylacetat extrahiert, die vereinigten organischen Phasen über Natriumsulfat/Aktivkohle getrocknet und eingedampft. Das rohe Öl wird säulenchromatographisch gereinigt (1 : 40, Kieselgel 60, Korngröße 0,040 bis 0,063 ; Eluens: Ethylacetat : Petrolether = 3 : 1).
Ausbeute : 0,52 g gelbliche Kristalle (26,5% d.Th.)
Fp = 67 - 68°C (Diisopropylether)

Das Ausgangsmaterial kann wie folgt hergestellt werden :

4-Hydroxy-2-thiophencarbonsäuremethylester (XI)

50,0 g (0,347 mol) 4-Hydroxy-2-thiophencarbonsäure und 58,3 g (0,694 mol) Natriumbicarbonat werden in 990 ml absolutem 2-Butanon unter Stickstoff zum Sieden erhitzt und 43,7 g (0,347 mol) Dimethylsulfat innerhalb von 20 Minuten zugetropft. Es wird noch 2,5 Stunden unter Rückfluß erhitzt. Anschließend wird im Vakuum eingedampft und der Rückstand zwischen gesättigter Natriumbicarbonatlösung und Ether verteilt. Die wäßrige Phase wird noch fünfmal mit je 80 ml Ether extrahiert. Die vereinigten organischen Phasen werden über Natriumsulfat/Aktivkohle getrocknet, filtriert und eingedampft.
Ausbeute : 49,6 g gelbliche Kristalle (90%)
Fp = 84 - 85°C (Diisopropylether/Petrolether)

5-(7-Jodheptyl)-3-methyl-isoxazol (IX)

16,66 g (77,23 mmol) 5-(7-Chlorheptyl)-3-methyl-isoxazol (VIII) und 12,75 g (85,06 mmol) Natriumjodid NaJ werden in 110 ml wasserfreiem Aceton unter Rückfluß erhitzt. Nach 7 h zeigt ein $^1$H-NMR etwa 85 %, nach 22 h etwa 89 % Umsatz. Nach 27 h wird eingeengt und zwischen Dichlormethan und Wasser (unter Zugabe von einigen ml 2n Salzsäure) verteilt. Die wäßrige Phase wird mehrmals mit insgesamt 250 ml Dichlormethan ausgeschüttelt und die organische Phase über Natriumsulfat getrocknet und eingedampft.
Ausbeute : 22,95 g braune Flüssigkeit (96,7% d.Th.)

4-(7-(3-Methyl-5-isoxazolyl)-heptyloxy)-2-thiophencarbonsäuremethylester(XIIa)

6,42 g (40,60 mmol) 4-Hydroxy-2-thiophencarbonsäuremethylester (XI) und 11,88 g (38,67 mmol) 5-(7-Jodheptyl)-3-methyl-isoxazol (IX) werden mit 5,34 g (40,60 mmol) Kaliumkarbonat ($K_2CO_3$) in 130 ml wasserfreiem Aceton 8 Stunden unter Rückfluß erhitzt. Nach Stehen über Nacht wird eingeengt, zwischen 2n NaOH und Ether verteilt und die wäßrige Phase mehrmals mit insgesamt 150 ml Ether extrahiert. Die organische Phase wird über Natriumsulfat/Aktivkohle getrocknet und eingedampft.
Ausbeute : 12,56 g gelbe Kristalle (96,3% d.Th.)
Fp. = 58-60°C

4-(7-(3-Methyl-5-isoxazolyl)-heptyloxy)-2-thiophencarbonsäure (XIII)

11,34 g (33,61 mmol) 4-(7-(3-Methyl-5-isoxazolyl)-heptyloxy)-2-thiophencarbonsäuremethylester werden in 95 ml Ethanol und 45 ml Wasser unter Rückfluß erhitzt und in diese Lösung 2,18 g (38,9 mmol) KOH, gelöst in 70 ml Wasser und 46 ml Ethanol, zugetropft. Nach 3 h Erhitzen unter Rückfluß wird abgekühlt, eingeengt, der Rückstand zwischen Wasser und Ether verteilt und die wässrige Phase nach Ansäuern mit 2n HCl auf pH-Wert 1 mehrmals mit Ether extrahiert. Die vereinigten organischen Phasen werden über Natriumsulfat/Aktivkohle getrocknet und eingedampft.
Ausbeute : 9,28 g gelbe Kristalle (85,6% d.Th.)
Das Rohprodukt kann direkt in der nächsten Stufe eingesetzt, oder aus Diisopropylether umkristallisiert werden, wodurch farblose Kristalle erhalten werden.
Fp. = 110-113°C

N-(2-Hydroxyethyl)-4-(7-(3-methyl-5-isoxazolyl)-heptyloxy)-2-thiophencarbonsäureamid (IV)

Zu 5,3 g (16,44 mmol) 4-(7-(3-Methyl-5-isoxazolyl)-heptyloxy)-2-thiophencarbonsäure werden bei 0°C ca. 15 ml Thionylchlorid zugegeben und 20 min. bei Raumtemperatur gerührt. Nach Absaugen am Wasserstrahlvakuum wird der Rückstand in 40 ml wasserfreiem Dichlormethan aufgenommen und eine Lösung von 2,2 g (36,09 mmol) Ethanolamin in 40 ml wasserfreiem Dichlormethan unter Kühlung zugetropft. Nach 2-stündigem Rühren bei Raumtemperatur wird abgekühlt, zwischen Wasser und Dichlormethan verteilt, wobei zur besseren Phasentrennung etwas 2n HCl zugegeben wird. Nach Rückschütteln beider Phasen wird die organische Phase über Natriumsulfat/Aktivkohle getrocknet und eingedampft.
Ausbeute : 5,74 g braune Kristalle (95,6% d.Th.)
Fp. : 56 - 57°C (Rohprodukt, kann ohne Ausbeuteverluste direkt zur Herstellung der Verbindung der Formel I verwendet werden).

**Ansprüche**

Patentansprüche für die Vertragsstaaten :BE,CH,DE,FR,GB,IT,LI,LU,NL,SE

1. Substituierte Isoxazolderivate der allgemeinen Formel (I)

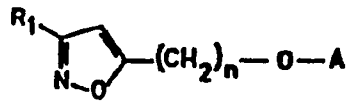

I

in der
$R_1$ $(C_1 - C_4)$-Alkyl
n eine ganze Zahl 6, 7 oder 8,
A eine Gruppe der allgemeinen Formeln (II) und (III)

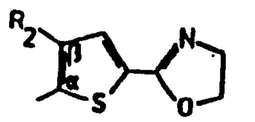

II oder

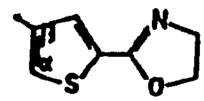

III

und $R_2$ Wasserstoff oder Methyl
bedeuten.

2. Verbindungen der allgemeinen Formel (I) nach Anspruch 1, worin A eine Gruppe der allgemeinen Formel (II) bedeutet.

3. Verbindungen der allgemeinen Formel (I) nach einem der Ansprüche 1 oder 2, worin $R_1$ Methyl und $R_2$ Wasserstoff bedeutet.

4. Die Verbindung nach Anspruch 1, 5-(7-(5-(4,5-Dihydro-2-oxazolyl)-2-thienyl)oxyheptyl)-3-methylisoxaxol.

5. Die Verbindung nach Anspruch 1, 5-(7-(2-(4,5-Dihydro-2-oxazolyl)-4-thienyl)oxyheptyl)-3-methylisoxaxol.

6. Verfahren zur Herstellung von Verbindungen gemäß Anspruch 1, dadurch gekennzeichnet, daß man eine Verbindung der allgemeinen Formel (IV)

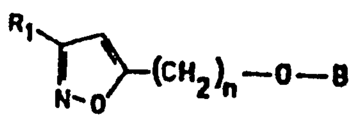

IV

in der
$R_1$ und n wie in oben definiert sind,
B eine Gruppe der allgemeinen Formeln (V) oder (VI)

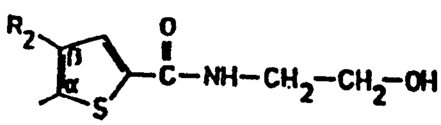

V

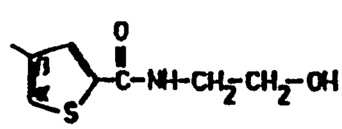

VI

bedeutet und
$R_2$ wie oben definiert ist, durch Behandeln mit einem wasserentziehenden Reagens unter Bildung des Oxazolrings cyclisiert.

7. Pharmazeutische Präparate, enthaltend Verbindungen gemäß Anspruch 1 in Kombination mit

üblichen galenischen Hilfs- und/oder Trägerstoffen.

8. Pharmazeutische Präparate, enthaltend Verbindungen gemäß Anspruch 1 in Kombination mit anderen antiinfektiven oder antiviralen Wirkstoffen sowie üblichen galenischen Hilfs- und/oder Trägerstoffen.

9. Verwendung der Verbindungen gemäß Anspruch 1 zur Herstellung von Arzneimitteln zur Behandlung und Prophylaxe von Viruserkrankungen.

Patentansprüche für den Vertragssstaat :AT

1. Verfahren zur Herstellung neuer substituierter Isoxazolderivate der allgemeinen Formel (I)

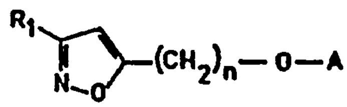

I

in der

$R_1$ $(C_1 - C_4)$-Alkyl,

n eine ganze Zahl 6, 7 oder 8,

A eine Gruppe der allgemeinen Formel)(II) oder (III)

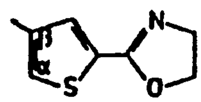

III

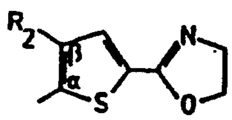

II

und

$R_2$ Wasserstoff oder Methyl bedeuten, dadurch gekennzeichnet, daß man eine Verbindung der allgemeinen Formel (IV)

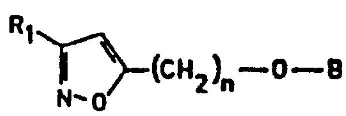

IV

in der

$R_1$ und n wie oben definiert sind,

B eine Gruppe der allgemeinen Formel (V) oder (VI)

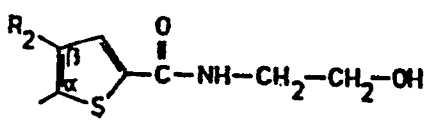

V

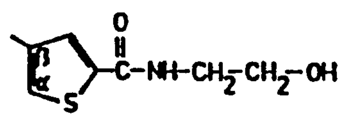

VI

bedeutet und

$R_2$ wie oben definiert ist, durch Behandeln mit einem wasserentziehenden Reagens unter Bildung des Oxazolrings cyclisiert.

2. Verfahren nach Anspruch 1, dadurch gekennzeichnet, daß man eine Verbindung der gemeinen Formel (IV), worin B eine Gruppe der Formel (V) bedeutet, cyclisiert.

3. Verfahren nach einem der Ansprüche 1 oder 2, dadurch gekennzeichnet, daß man eine Verbindung

der allgemeinen Formel (IV), worin $R_1$ Methyl und $R_2$ Wasserstoff bedeutet, cyclisiert.

4. Verfahren nach Anspruch 1, dadurch gekennzeichnet, daß man eine Verbindung der allgemeinen Formel (IV) worin $R_1$ Methyl, n die ganze Zahl 7, B eine Gruppe der Formel (V) und $R_2$ Wasserstoff bedeuten, cyclisiert.

5. Verfahren nach Anspruch 1, dadurch gekennzeichnet, daß man eine Verbindung der allgemeinen Formel (IV), worin $R_1$ Methyl, n die ganze Zahl 7 und B eine Gruppe der Formel (VI) bedeuten, cyclisiert.

6. Verfahren nach einem der Ansprüche 1 bis 5, dadurch gekennzeichnet, daß man die Cyclisierung in einem inerten Lösungsmittel in Gegenwart von Phosphoroxychlorid, Phosphorpentachlorid oder Thionylchlorid bei -5°C bis +5° C durchführt.

7. Verfahren nach Anspruch 6, dadurch gekennzeichnet, daß man das wasserentziehende Reagens in einem 1.1 bis 3-fachen molaren Überschuß pro Mol der Verbindung der Formel (IV) einsetzt.

8. Verfahren nach einem der Ansprüche 1 bis 5, dadurch gekennzeichnet, daß man die Cyclisierung in einem Überschuß eines flüssigen wasserentziehenden Reagens ohne gesondertes Lösungsmittel bei -15 bis +5°C durchführt.

9. Verfahren nach Anspruch 8, dadurch gekennzeichnet, daß man zur Cyclisierung Thionylchlorid verwendet.

10. Verfahren zur Herstellung von pharmazeutischen Präparaten, dadurch gekennzeichnet, daß man Verbindungen der allgemeinen Formel I, wie sie gemäß Anspruch 1 definiert sind, mit für die orale oder parenterale Applikation geeigneten, pharmazeutischen, organischen oder anorganischen Hilfs- und/oder Trägerstoffen und gegebenenfalls anderen antiinfektiven oder antiviralen Wirkstoffen zu festen, halbfesten oder flüssigen Arzneiformen verarbeitet.

## Claims

Claims for the Contracting States : BE, CH, DE, FR, GB, IT, LI, LU, NL, SE,

1. Substituted isoxazole derivatives of the formula (I)

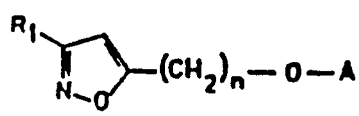

I

in which
$R_1$ denotes $(C_1\text{-}C_4)$-alkyl,
n denotes an integer 6, 7 or 8,
A denotes a group of the formulae (II) and (III)

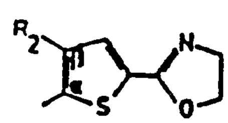

II or

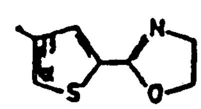

III

and
$R_2$ denotes hydrogen or methyl.

2. Compounds of the formula (I) according to Claim 1, in which A denotes a group of the formula (II).

3. Compounds of the formula (I) according to either of Claims I and 2, in which $R_1$ denotes methyl and $R_2$ denotes hydrogen.

4. The compound 5-(7-(5-(4,5-dihydro-2-oxazolyl)-2thienyl)oxyheptyl)-3-methyl-isoxazole according to Claim 1.

5. The compound 5-(7-(2-(4,5-dihydro-2-oxazolyl)-4thienyl)oxyheptyl)-3-methyl-isoxazole according to Claim 1.

6. Process for the preparation of compounds according to Claim 1, characterized in that a compound of the formula (IV)

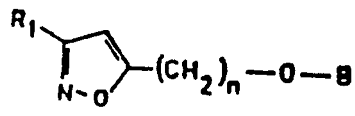

IV

in which
$R_1$ and n are as defined above,
B denotes a group of the formulae (V) or (VI)

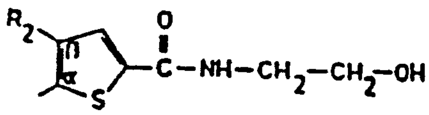

V

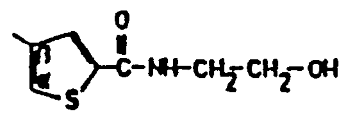

VI

and
$R_2$ is as defined above, is cyclized by treatment with a dehydrating reagent, to form the oxazole ring.

7. Pharmaceutical products containing compounds according to Claim 1 in combination with customary pharmaceutical auxiliaries and/or excipients.

8. Pharmaceutical products containing compounds according to Claim 1 in combination with other anti-infective or antiviral active compounds and customary pharmaceutical auxiliaries and/or excipients.

9. Use of the compounds according to Claim 1 for the preparation of medicaments for the treatment and prophylaxis of virus diseases.

Claims for the Contracting State :AT

1. Process for the preparation of novel substituted isoxazole derivatives of the formula (I)

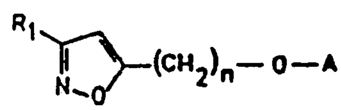

I

in which
$R_1$ denotes $(C_1\text{-}C_4)$-alkyl,
n denotes an integer 6, 7 or 8,
A denotes a group of the formula (II) or (III)

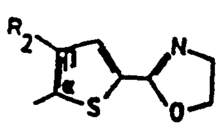

II

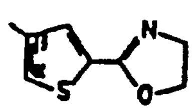

III

and
$R_2$ denotes hydrogen or methyl, characterized in that a compound of the formula (IV)

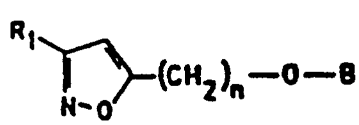

IV

in which
$R_1$ and n are as defined above,

B denotes a group of the formula (V) or (VI)

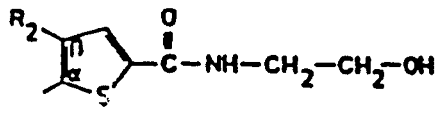
V

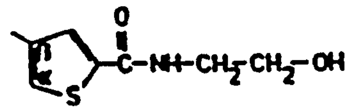
VI

and
$R_2$ is as defined above, is cyclized by treatment with a dehydrating reagent, to form the oxazole ring.

2. Process according to Claim 1, characterized in that a compound of the formula (IV) in which B denotes a group of the formula (V) is cyclized.

3. Process according to either of Claims 1 and 2, characterized in that a compound of the general formula (IV) in which $R_1$ denotes methyl and $R_2$ denotes hydrogen is cyclized.

4. Process according to Claim 1, characterized in that a compound of the general formula (IV) in which $R_1$ denotes methyl, n denotes the integer 7, B denotes a group of the formula (V) and $R_2$ denotes hydrogen is cyclized.

5. Process according to Claim I, characterized in that a compound of the general formula (IV) in which $R_1$ denotes methyl, n denotes the integer 7 and B denotes a group of the formula (VI) is cyclized.

6. Process according to any one of Claims 1 to 5, characterized in that the cyclization is carried out in an inert solvent in the presence of phosphorus oxychloride, phosphorus pentachloride or thionyl chloride at -5°C to +5°C.

7. Process according to Claim 6, characterized in that the dehydrating agent is used in a 1.1- to 3-fold molar excess per mol of the compound of the formula (IV).

8. Process according to any one of Claims 1 to 5, characterized in that the cyclization is carried out in an excess of a liquid dehydrating reagent without a separate solvent, at -15 to +5°C.

9. Process according to Claim 8, characterized in that thionyl chloride is used for the cyclization.

10. Process for the preparation of pharmaceutical products, characterized in that compounds of the general formula I, as they are defined according to Claim 1, are processed with pharmaceutical, organic or inorganic auxiliaries and or excipients suitable for oral or parenteral administration and, if appropriate, other anti-infective or antiviral active compounds, to give solid, semi-solid or liquid medicament forms.

**Revendications**

POUR LES ETATS CONTRACTANTS :BE,CH,DE,FR,GB,IT,LI,LU,NL,SE

1. Dérivés substitués isoxazoles de formule générale (I) :

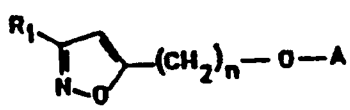
I

dans laquelle
$R_1$ est un groupe alkyle de 1 à 4 atomes de carbone,
n un nombre entier égal à 6, 7 ou 8,
A représente un groupe de formule générale (II) ou (III) :

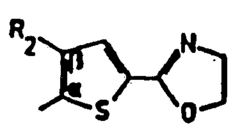

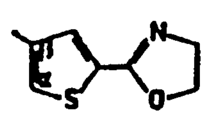
II ou III

et
$R_2$ représente l'hydrogène ou un groupe méthyle.

2. Composés de formule générale (I) selon la revendication 1, dans lesquels A signifie un groupe de formule générale (II).

3. Composés de formule générale (I) selon l'une des revendications 1 ou 2, dans lesquels $R_1$ représente un groupe méthyle et $R_2$ l'hydrogène.

4. Composé selon la revendication 1, constitué par le 5-(7-(5-(4,5-dihydro-2-oxazolyl)-2-thiényl)oxyheptyl)-3-méthyl-isoxazole.

5. Composé selon la revendication 1, constitué par le 5-(7-(2-(4,5-dihydro-2-oxazolyl)-4-thiényl))oxyheptyl)-3-méthylisoxazole.

6. Procédé de préparation des composés selon la revendication 1, caractérisé en ce qu'on réalise une cyclisation d'un composé de formule générale (IV) :

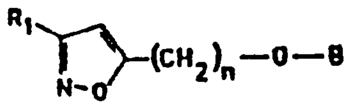

IV

dans laquelle
$R_1$ et n sont définis comme ci-dessus,
B représente un groupe de formule générale (V) ou (VI) :

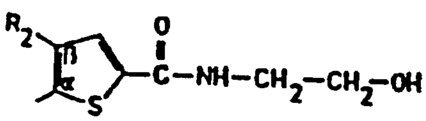

V ou

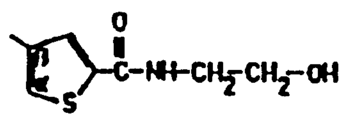

VI

et $R_2$ est défini comme ci-dessus, par action d'un réactif éliminant l'eau et formation du cycle oxazole.

7. Préparation pharmaceutique renfermant les composés selon la revendication 1 en combinaison avec les adjuvants et/ou substances vectrices galéniques.

8. Préparations pharmaceutiques renfermant les composés selon la revendication 1 en combinaison avec d'autres substances à effet anti infectieux et antiviral ainsi que les adjuvants et substances vectrices galéniques habituelles.

9. Utilisation des composés selon la revendication 1, pour la préparation de médicaments dans le traitement et la prophylaxie des maladies virales.

POUR L'ETAT CONTRACTANT :AT

1. Procédé de préparation de nouveaux dérivés isoxazoles substitués de formule générale (I) :

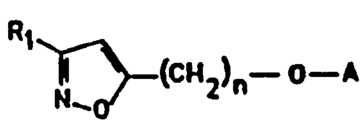

I

dans laquelle
$R_1$ représente un groupe alkyle de 1 à 4 atomes de carbone,
n un nombre entier égal à 6, 7 ou 8,
A un groupe de formule générale (II) ou (III) :

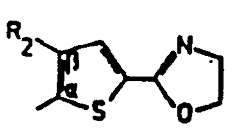

II ou

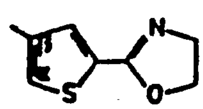

III

et
$R_2$ l'hydrogène ou un groupe méthyle, caractérisé en ce qu'on réalise une cyclisation d'un composé de formule générale (IV) :

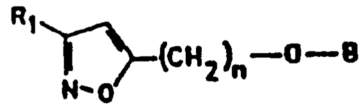

IV

dans laquelle
$R_1$ et n sont définis comme ci-dessus,
B représente un groupe de formule générale (V) ou (VI)

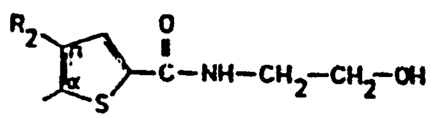

V

ou

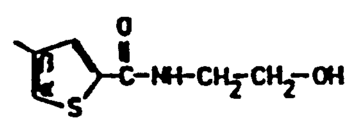

VI

et
$R_2$ est défini comme ci-dessus, par action d'un réactif éliminant l'eau et formation du cycle oxazole.

2. Procédé selon la revendication 1, caractérisé en ce qu'on réalise une cyclisation d'un composé de formule (IV) dans lequel B est un groupe de formule (V).

3. Procédé selon l'une des revendications 1 ou 2, caractérisé en ce qu'on réalise une cyclisation d'un composé de formule générale (IV), dans lequel $R_1$ représente un groupe méthyle et $R_2$ l'hydrogène.

4. Procédé selon la revendication 1, caractérisé en ce qu'on réalise une cyclisation d'un composé de formule générale (IV) dans lequel $R_1$ est un groupe méthyle, n est égal à 7, B représente un groupe de formule (V) et $R_2$ l'hydrogène.

5. Procédé selon la revendication 1, caractérisé en ce qu'on réalise une cyclisation du composé de formule (IV) dans lequel $R_1$ est un groupe méthyle, n est égal à 7, B représente un groupe de formule (VI)

6. Procédé selon une des revendications 1 à 5, caractérisé en ce qu'on réalise la cyclisation dans un solvant inerte en présence d'oxychlorure de phosphore, de pentachlorure de phosphore ou de chlorure de thionyle à une température comprise entre -5 et +5°C

7. Procédé selon la revendication 6, caractérisé en ce que le réactif d'élimination de l'eau est utilisé en un excès molaire de 1,1 à 3 fois par mole du composé de formule (IV).

8. Procédé selon l'une des revendications 1 à 5, caractérisé en ce qu'on réalise la cyclisation dans un excès de réactif liquide d'élimination de l'eau sans solvant particulier à une température comprise entre -15 et +5°C.

9. Procédé selon la revendication 8, caractérisé en ce que la cyclisation est obtenue avec le chlorure de thionyle.

10. Procédé de préparation de compositions pharmaceutiques, caractérisé en ce qu'on utilise des composés de formule générale (I) tels que définis dans la revendication 1 avec des adjuvants et/ou des substances vectrices organiques ou non, pharmaceutiques, utilisables dans une administration orale ou parentérale et le cas échéant avec d'autres principes actifs anti-infectieux et antiviraux pour préparer des formules médicamenteuses solides, semisolides ou liquides.